# EUROPEAN PATENT APPLICATION

(11) **EP 1 788 382 A1**
(43) Date of publication of application: **23.05.2007**
(21) Application number: 06024071.0
(22) Date of filing: 20.11.2006
(51) Int. Cl.: G01N 27/30, G01N 27/49

(54) **Electrochemical residual chlorine measuring method and device with a doped diamond working electrode**

(30) Priority: 22.11.2005 JP 2005337769
(71) Applicant: HORIBA, LTD., Kyoto-shi, Kyoto (JP); Keio University, Tokyo 108-8345 (JP)
(72) Inventor: Nomura, Satoshi, Minami-ku Kyoto-city Kyoto (JP); Einaga, Yasuaki Keio University, Kohoku-ku Yokohama-shi Kanagawa (JP); Matsumoto, Koichi, Minami-ku Kyoto-city Kyoto (JP); Murata, Michio Keio University, Kohoku-ku Yokohama-shi Kanagawa (JP); Shibata, Mamoru Keio University, Kohoku-ku Yokohama-shi Kanagawa (JP)
(74) Representative: Müller - Hoffmann & Partner

(57) **Abstract**

A residual chlorine measuring method includes the steps of: bringing a counter electrode 4, a working electrode 2 and a reference electrode 3 into contact with a sample solution containing residual chlorine; applying a voltage between the counter electrode 4 and the working electrode 2; and measuring a current value when applying the voltage to calculate a concentration of the residual chlorine. The working electrode 2 is an electrically conductive diamond electrode to which boron is doped. The reference electrode 3 is a silver/silver chloride electrode. A current value when setting the potential of the electrically conductive diamond electrode 2 to the silver/silver chloride electrode 3 to +0.5 V to +1.5 V is measured.

## Description

### Field of the Invention

The present invention relates to a residual chlorine measuring method and device and more particular to method and to a device for measuring a residual chlorine concentration using an electrochemical method.

### Description of the Background Art

There has been a colorimetric assay method using a reagent such as a DPD method and an orthotolidine method and a polarographic method using an electrode as a method for measuring residual chlorine in a sample solution.

In the DPD method, a measurer compares pinkish red color produced by reacting the residual chlorine with diethyl-p-phenylenediamine (DPD) with a color chart to determine the concentration of the residual chlorine. In the orthotolidine method, the measurer compares yellow color produced by reacting the residual chlorine with a chloride solution of orthotolidine with the color chart to determine the concentration of the residual chlorine.

However, since these methods are based on the visual judgment of the measurer, there is a problem in that individual difference arises is in the measured value. There is also a problem in that waste liquid treatment is required after the measurement. Furthermore, there is also a problem in that the cost for the preparation of the reagent is high.

On the other hand, the polarographic method using the electrode determines the concentration of the residual chlorine by a current between a counter electrode and a working electrode. Since this method does not require the reagent, the waste liquid treatment is not required, and furthermore, the residual chlorine is easily measured.

However, since the conventional polarographic method uses a platinum electrode for the working electrode as shown in Patent Reference 1, there is a problem in that an oxidation current peak of the residual chlorine appears only in the vicinity of the limit of a potential window, and overlaps the potential window, thereby disturbing the exact measurement.
Patent document 1: Japanese Examined Patent Publication No. 1980-17939

### Summary of the invention

Then, the present invention has been developed to eliminate the above conventional problems described above easily. It is a main desired object of the present invention to provide an objective measured result without using a harmful reagent and furthermore and to correctly and easily measure the residual chlorine without being influenced by the potential window.

The object underlying the present invention is achieved by a chlorine measuring method according to independent claim 1 and and by a chlorine measuring device according to independent claim 5. Preffered embodiments are within the scope of the respective dependent claims.

That is, a residual chlorine measuring method according to the present invention, includes the steps of: bringing a counter electrode, a working electrode and a reference electrode into contact with a sample solution containing residual chlorine which is an object to be measured; applying a voltage between the counter electrode and the working electrode; and measuring a current value under the voltage to calculate a concentration of the residual chlorine, wherein the working electrode is an electrically conductive diamond electrode to which a group 13 element or a group 15 element is doped; the reference electrode is a silver/silver chloride electrode; and a current value is measured when a potential of the electrically conductive diamond electrode is set to +0,5 V to +1,5 V when compared to a potential of the silver/silver chloride electrode.

Herein, the set of the potential of the electrically conductive diamond electrode to the silver/silver chloride electrode to +0,5 V to +1,5 V is based on that the peak of a current produced by an oxidation reaction of the residual chlorine is generated between +0,5 V and +1,5 V and the oxidation-reduction reaction of the residual chlorine is hardly produced less than +0,5 V.

In the present invention, the residual chlorine means all available chlorines which remain in water, and includes two kinds, that is, free residual chlorine and bonding residual chlorine. The free residual chlorine is chlorine (Cl₂), hypochlorous acid (HClO) and hypochlorous acid ion (ClO⁻). The bonding residual chlorine is monochloramine (NH₂Cl), dichloramine (NHCl₂) and trichloramine (NCl₃).

The residual chlorine measuring method can provide the objective measured result without using the harmful reagent. Also, since the electrically conductive diamond electrode to which the group 13 element or the group 15 element is doped has an advantageous character in which a potential window (an oxidation potential and an reduction potential are wide) is wide and a background current (a residual current) is lower than those of the other electrode materials, the concentration of the residual chlorine can be highly sensitively, highly precisely and easily measured. Furthermore, the potential of the electrically conductive diamond electrode to the silver/silver chloride electrode is changed only within the range of +0,5 V to +1,5V where the peak of the current due to the oxidation reaction of the residual chlorine is generated and it is not necessary to measure the potential less than +0,5 V. Thereby, it is possible to measure the concentration of the residual chlorine for a short time.

The group 13 element or the group 15 element is preferably at least one kind of element selected from the group consisting of boron, nitrogen and phosphorus, and particularly preferably a boron-doped diamond electrode into which the boron is mixed.

Although examples of the surface states of the above electrically conductive diamond electrode to which the boron is doped include a hydrogen-terminated surface state and an oxygen-terminated surface state, the hydrogen-terminated surface state is more preferable.

The reason for this is the following. That is, in the electrically conductive diamond electrode oxygen-terminated, the oxidation potential corresponding to the detected peak current is at a higher potential side, and the peak current comes near the potential window, and thereby the sensitivity may be reduced. However, in the electrically conductive diamond electrode hydrogen-terminated, the oxidation potential corresponding to the detected peak current is at a lower potential side as compared with a case of using the electrically conductive diamond electrode oxygen-terminated and the peak current is separately detected from the potential window, thereby further enhancing the sensitivity.

Also, as a specific method for oxygen-terminating, it is desirable to anodize the electrically conductive diamond electrode or subject the electrically conductive diamond electrode to oxygen plasma treatment to oxygen-terminate the electrically conductive diamond electrode. As a specific method for hydrogen-terminating the electrically conductive diamond electrode, it is desirable to anneal (heat) the electrically conductive diamond electrode under a hydrogen atmosphere or cathodically reduce the electrically conductive diamond electrode to hydrogen-terminate the electrically conductive diamond electrode.

Also, the residual chlorine measuring method according to the present invention can be carried out by, for example, a measuring device having the following constitution. That is, a residual chlorine measuring device for measuring a residual chlorine concentration in a sample solution, including: a working electrode; a counter electrode; a reference electrode; a voltage applying part for applying a voltage to the working electrode and the counter electrode; a current measuring part for measuring a current value in the applied voltage; and an information processor for calculating a residual chlorine concentration based on a current measuring signal from the current measuring part, wherein the working electrode is an electrically conductive diamond electrode to which a group 13 element or a group 15 element is doped; the reference electrode is a silver/silver chloride electrode; and the information processor controls the potential of the electrically conductive diamond electrode to the silver/silver chloride electrode to +0,5 V to +1,5 V.

Thus, the present invention can provide the objective measured result without using the harmful reagent.

Also, since the electrically conductive diamond electrode to which the group 13 element or the group 15 element is doped has an advantageous character in which a potential window (an oxidation potential and an reduction potential are wide) is wide and a background current (a residual current) is lower than those of the other electrode materials, the concentration of the residual chlorine can be highly sensitively, highly precisely and easily measured.

Furthermore, the potential of the electrically conductive diamond electrode to the silver/silver chloride electrode is changed only within the range of +0,5 V to + 1,5V where the peak of the current due to the oxidation reaction of the residual chlorine is generated and it is not necessary to measure the potential less than +0,5 V.

Thereby, it is possible to measure the concentration of the residual chlorine for a short time.

### Brief description of the drawings

- **FIG. 1**: is a schematic constitution diagram of a residual chlorine measuring device according to a first embodiment of the present invention.
- **FIG. 2**: is voltammogram for each of the concentrations of residual chlorines when linearly sweeping a potential of a working electrode between +0.5 V and +1.5 V in the embodiment.
- **FIG. 3**: shows a calibration curve produced based on the current-voltage curve shown in FIG. 2.
- **FIG. 4**: is a schematic constitution diagram of a residual chlorine measuring device according to a second embodiment of the present invention.
- **FIG. 5**: shows a current value of each of the concentrations of residual chlorines when a potential of a working electrode is +1.1 V in the embodiment.
- **FIG. 6**: shows a calibration curve produced based on the measured results shown in FIG. 5.

### Description of preferred embodiments

### <First Embodiment>

Hereinafter, a first embodiment of a residual chlorine measuring device according to the present invention will be described referring to the drawings.

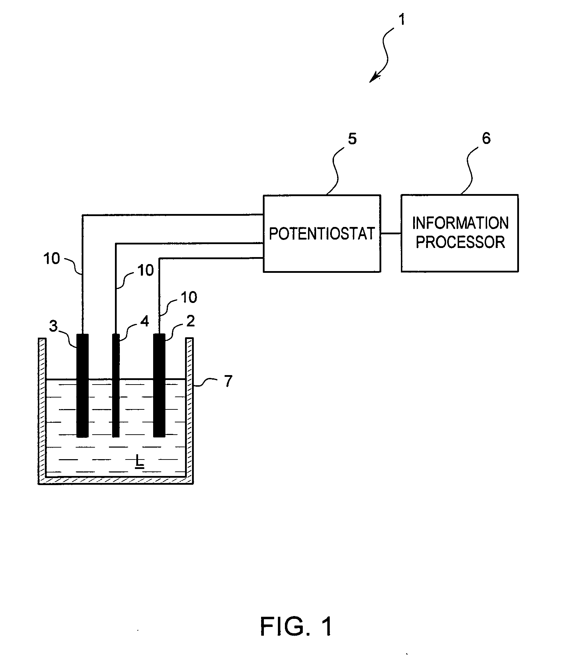

A residual chlorine measuring device 1 according to the present embodiment is a batch-type electrochemistry measuring device which analyzes a sample solution L by dissolving an electrolyte in the sample solution L to produce an electrolyte solution and then by applying a voltage to the solution and performs voltammetry measurement for analyzing the solution L due to a triple electrode system. As shown in FIG. 1, the basic constitution includes a working electrode 2, a reference electrode 3, a counter electrode 4, a potentiostat 5 for controlling the voltages of the working electrode 2, the reference electrode 3 and the counter electrode 4, and an information processor 6 for calculating, for example, the concentration of residual chlorine contained in the sample solution L based on a current and voltage obtained by the potentiostat 5.

The sample solution L contains the residual chlorine which is the object to be measured, and the embodiment uses hypochlorite. Also, sodium perchlorate (NaClO₄) of 0,1 mol/l is used as the electrolyte.

The working electrode 2 applies a voltage to the sample solution, and is a boron-doped diamond electrode to which conductivity is imparted by adding boron with high density. Also, the working electrode 2 is fixed by a holding member which is not shown so as to be immersed in the sample solution L accommodated in a measuring cell 7. The potential to the reference electrode 3 is swept between +0,5 V and +1,5 V by the information processor 6 to be described later. The boron-doped diamond electrode 1 to which boron is doped at high concentration has an advantageous character in which a potential window is wide (an oxidation potential and a reduction potential are wide) and a background current is lower than those of the other electrode materials. Also, the boron-doped diamond electrode 1 is excellent in chemical resistance, durability, electrical conductivity and corrosion resistance or the like.

The boron-doped diamond electrode used in the present invention is produced by forming a diamond film on an upper surface of a silicon substrate by, for example, using a microwave plasma CVD method. Now, boron is doped as impurities in order to make the diamond film conductive. A specific manufacturing method is as follows.

A silicon substrate (Si) is used as a substrate. A mixed solution of acetone (72 ml) and methanol (8 ml) is used as a carbon source. Boron oxide (B₂O₃) (1,09 g) is dissolved in the mixed solution. After this carbon source is bubbled by hydrogen gas, the carbon source is introduced into a chamber where a film is formed at a substrate temperature of about 800°C.

Although the addition amount of the boron to be mixed is suitably determined in the range capable of imparting conductivity to the diamond electrode 1, for example, the addition amount imparts the conductivity of about 1×10⁻² Ωcm to about 10⁻⁶ Ωcm to the diamond electrode 1.

The surface of the electrically conductive diamond electrode 1 thus formed is almost hydrogen-terminated in the "as grown" state (a surface treatment or the like is not then applied with a crystal grown on a substrate).

Then, is the electrically conductive diamond electrode 1 in the "as grown" state is cathodically reduced by applying the voltage of -1,8 V to the electrically conductive diamond electrode 1 and by immersing in a sulfuric acid (H₂SO₄) of 0,1 mol/l for 30 minutes to hydrogen-terminate the entire surface of the electrically conductive diamond electrode 1.

The reference electrode 3 is used as the standard of the potential of the working electrode 2, and the present embodiment uses a silver/silver chloride electrode (Ag/AgCl electrode). The reference electrode 3 is fixed by a holding member which is not shown so as to be immersed in the sample solution L accommodated in the measuring cell 7.

The counter electrode 4 makes a current flow in the working electrode 2 without trouble when setting the working electrode 2 to a potential, and is connected to the working electrode 2 in series. The present embodiment uses a platinum (Pt) electrode. The counter electrode 4 is also fixed by the holding member which is not shown so as to be immersed in the sample solution L accommodated in the measuring cell 7 as in the case of the reference electrode 3.

The potentiostat 5 functions as a voltage applying part for applying the voltage to the working electrode 2, the reference electrode and the counter electrode 4, and a current measuring part for measuring a current value in the applied voltage. The potentiostat 5 is controlled by the information processor 6 to be described later. The potentiostat 5 receives voltage signals and current signals from the working electrode 2, the reference electrode 3 and the counter electrode 4, and controls these electrodes 2, 3, 4. The potentiostat 5 always adjusts a voltage applied between the working electrode 2 and the counter electrode 4, and controls a voltage of the working electrode 2 to the reference electrode 3. Specifically, the potentiostat 5 scans at a rate of, for example, 100 mV/s while setting the potential of the working electrode 2 to the reference electrode 3 to +0,5 V to +1,5 V and detects a current value accompanying an oxidation reaction under the voltage.

The information processor 6 controls the potentiostat 5, determines a current-voltage curve based on voltage signals and current signals from the potentiostat 5, and calculates the concentration of the residual chlorine contained in the sample solution L based on the current-voltage curve. Furthermore, the information processor 6 controls the potentiostat 5 so as to change the potential of the working electrode 2 to the reference electrode 3 at a rate of, for example, 100 mV/s while setting the potential to +0,5 V to +1,5 V when measuring the concentration of the residual chlorine. Specifically, the information processor 6 has a CPU, an internal memory, an external storage such as a HDD, a communication interface such as a modem, a display, a mouse, and an input means such as a keyboard. The information processor 6 analyzes electric signals according to programs set in a predetermined region of the internal memory and the external storage or the like to detect of the residual chlorine and calculate the concentration thereof. The information processor 6 may be a generalized computer, and may be an exclusive use.

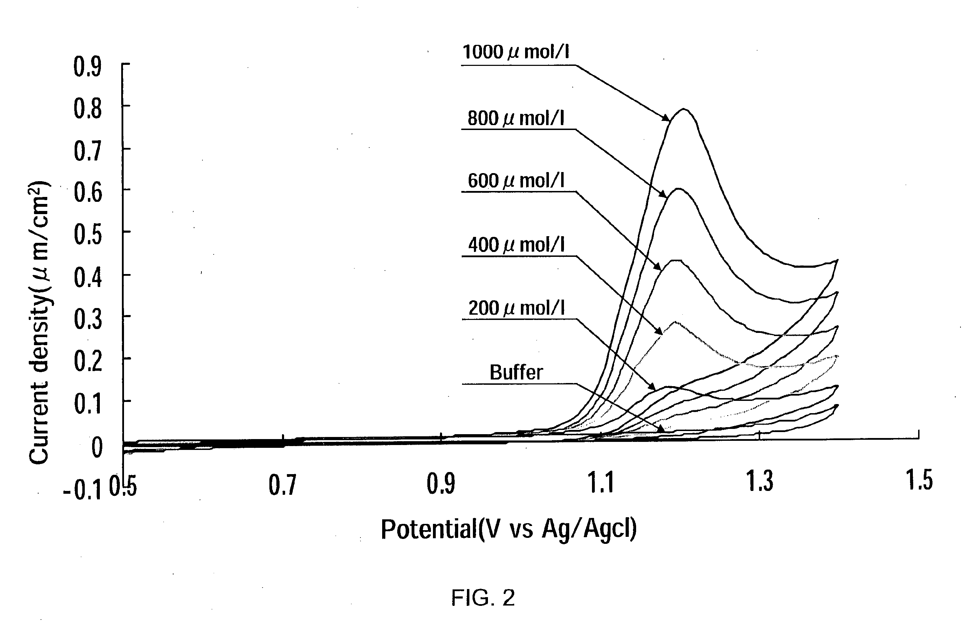

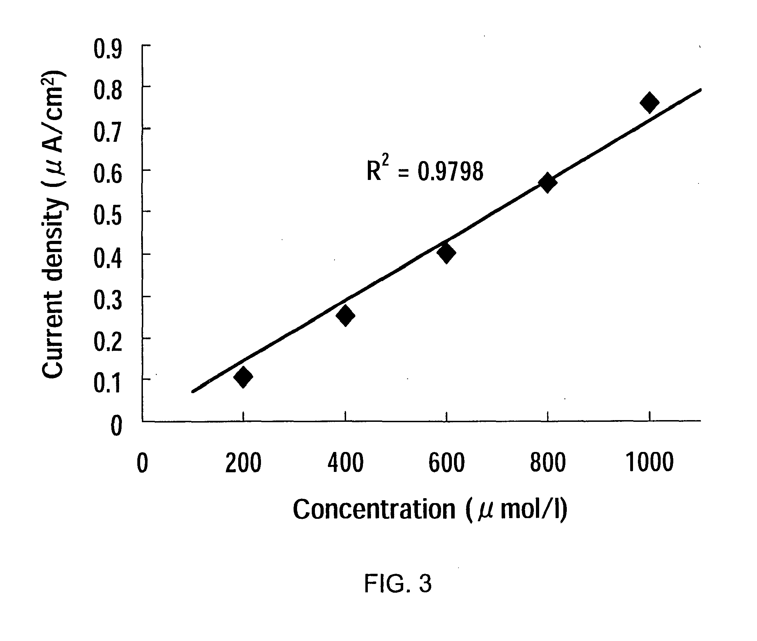

Next, FIGS. 2 and 3 show results obtained by measuring the residual chlorine contained in the sample solution L using the residual chlorine measuring device 1 according to the present embodiment.

FIG. 2 shows a current-voltage curve (voltammogram) obtained by measuring current values when linearly sweeping (100 mV/s) the potential of the working electrode 2 to the reference electrode 3 by the potentiostat 5 using the sample solutions L obtained by adjusting the concentration of the residual chlorine contained in the sample solutions L to 200 µmol/l, 400 µmol/l. 600 µmol/l, 800 µmol/l, 1000 µmol/l.

FIG. 3 shows a calibration curve of concentrations and current values in peak potentials in the vicinity of +1,2 V based on the results obtained in FIG. 2. As shown in FIG. 3, the calibration curve in which the concentration of the residual chlorine correlates with the current value well could be produced. Therefore, even a small amount (low concentration) of the residual chlorine can be correctly measured.

The residual chlorine measuring device 1 according to the present embodiment thus constituted can obtain an objective measured result without using a harmful reagent. Also, since the electrically conductive diamond electrode 2 to which the boron is doped has an advantageous character in which the potential window (the oxidation potential and the reduction potential are wide) is wide and the background current (a residual current) is lower than those of the other electrode materials, the concentration of the residual chlorine can be highly sensitively, highly precisely and easily measured. Furthermore, the potential of the electrically conductive diamond electrode 2 to the silver/silver chloride electrode 3 is changed only within the range of +0,5 V to +1,5V where the peak of the current due to the oxidation reaction of the residual chlorine is generated and it is not necessary to measure the potential less than +0,5 V. Thereby, it is possible to measure the concentration of the residual chlorine for a short time.

### <Second Embodiment>

Next, a second embodiment of the residual chlorine measuring device 1 according to the present invention will be described referring to the drawings. Elements corresponding to the first embodiment are designated by the same numerals.

The residual chlorine measuring device 1 according to the present embodiment performs a so-called flow injection analysis (FIA).

The flow injection analysis (FIA) produces continued flow controlled using a metering pump or the like, performs various reactions, a separation and a sample pouring or the like in this flow, and analyzes components contained in a solution using a detector provided with a flow cell provided at the end.

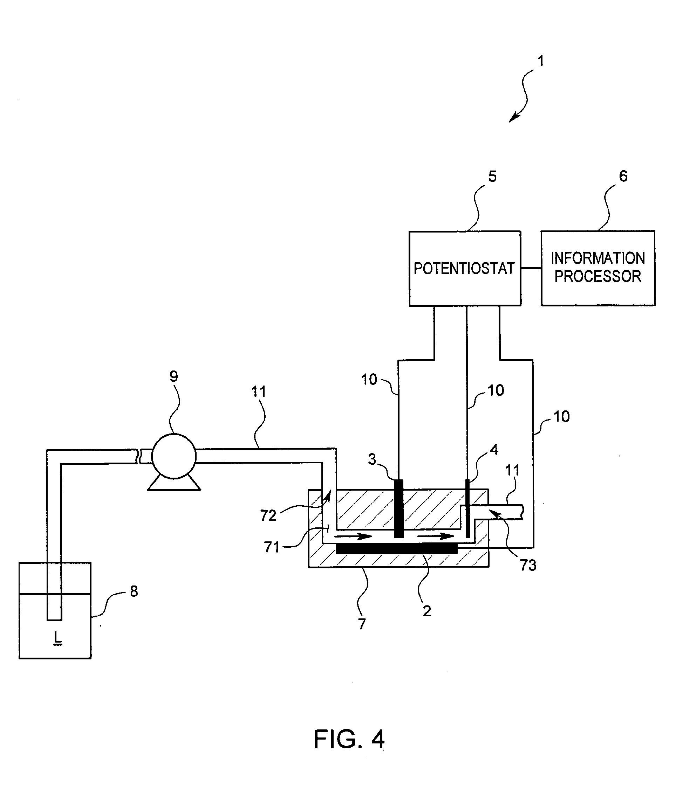

As shown in FIG. 4, the specific constitution of the device contains a flow cell 7 provided on a flow route of the sample solution L, the working electrode 2, the reference electrode 3 and the counter electrode 4 incorporated in the flow cell 7, the potentiostat 5 for controlling the voltages of the working electrode 2, the reference electrode 3 and the counter electrode 4, and an information processor 6 for calculating the concentration or the like of the residual chlorine in the sample solution L based on the current and the voltage obtained by the potentiostat 5.

The sample solution L contains the residual chlorine which is the object to be measured, and the present embodiment uses hypochlorite. Also, sodium perchlorate (NaClO₄) of 0,01 mol/l is used as the electrolyte.

The flow route is composed by a flow pipe 11 and the flow cell 7. The flow pipe 11 connects a solution tank 8 to an inflow port 72 of the flow cell 7, and connects an outflow port 73 of the flow cell 7 to a waste fluid tank (not shown). Also, a pump 9 is provided on a feed pipe 11 provided at an upstream side of the flow cell 7.

The flow cell 7 is constituted so that the electrically conductive diamond electrode 2, the reference electrode 3 and the counter electrode 4 are exposed in a flow passage 71 in which the sample solution L flows and can be brought into contact with the sample solution L. The diamond thin film of the electrically conductive diamond electrode 2 is exposed in the flow passage 71 and is brought into contact with the sample solution L. The sample solution L enters from the inflow port 72 of the flow passage 71, flows as shown by the arrow in FIG. 4, and reaches to the outflow port 73. An electrochemical reaction is generated in the sample solution L by applying a voltage between the working electrode 2 and the counter electrode 4.

The pump 9 is provided on the flow pipe 11 between the solution tank 8 and the flow cell 7, and can supply the sample solution L to the flow cell 7 at a fixed speed. For example, the pump 9 is a pump or the like for liquid chromatography.

Next, the operation of the residual chlorine measuring device 1 thus constituted will be described.

First, the sample solution L containing the residual chlorine which is the object to be measured is supplied to the flow cell 7 through the flow pipe 11 from the solution tank 8 by the pump 9. The electrochemical reaction is generated by applying the voltage between the working electrode 2 and the counter electrode 4 while the working electrode 2, reference electrode 3 and counter electrode 4 incorporated are brought into contact with the sample solution L in the flow cell 7. A current value (electric signals) produced by the electrochemical reaction is transmitted to the potentiostat 5, and the signals in each of the electrodes are controlled and detected. The signals detected by the potentiostat 5 are analyzed by the information processor 6, thereby detecting the residual chlorine and measuring the concentration thereof. The measured sample solution L is discharged out of the flow cell 7, and is accommodated in the waste fluid tank through the flow pipe 11.

Herein, the voltage applied between the working electrode 2 and the counter electrode 4 in the present embodiment is set to the voltage producing the maximum current value or the vicinity thereof in view of measurement efficiency and accuracy. Specifically, since the voltage which produces the maximum current value of the residual chlorine is about 1,2 V, the voltage applied between the working electrode 2 and the counter electrode 4 is set to about 1,1 V. Herein, the voltage which imparts the maximum current value imparts the maximum current value by, for example, cyclic voltammetry (CV). The voltage which imparts the maximum current value can be also determined by a rotating electrode method or a micro electrode method. The rotating electrode method or the micro electrode method is advantageous since the methods can further eliminate possibility of an error of measurement due to measurement conditions or the like.

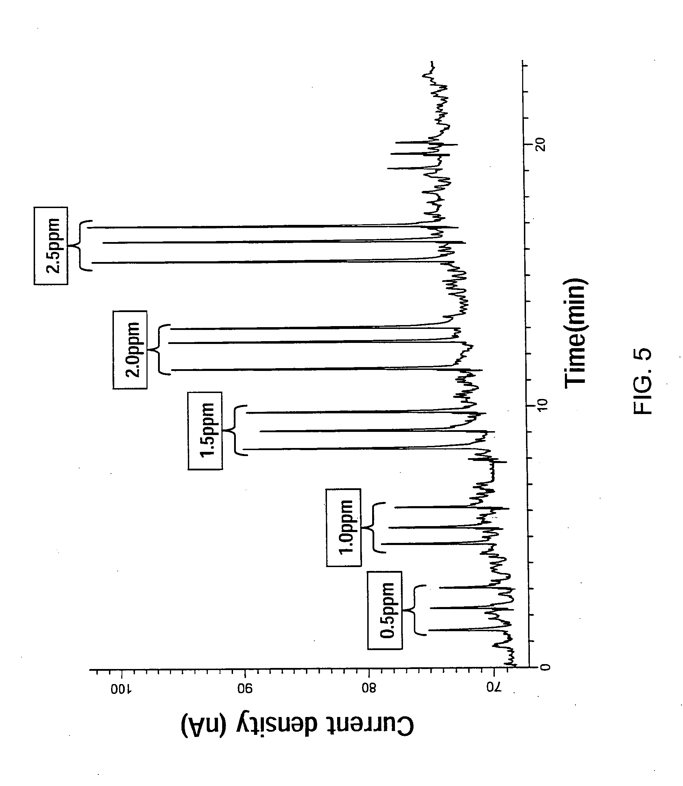

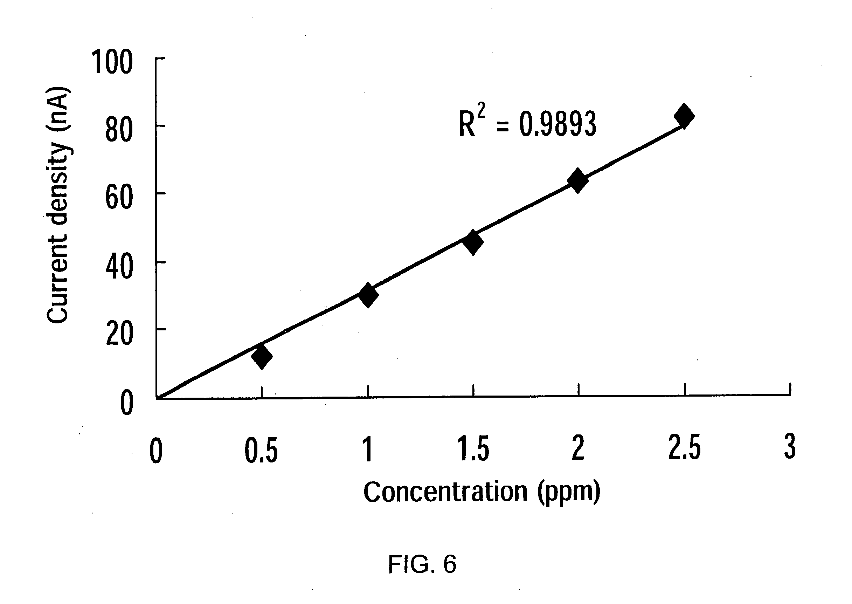

Next, FIGS. 5 and 6 show results obtained by measuring the residual chlorine contained in the sample solution using the residual chlorine measuring device 1 according to the present embodiment.

FIG. 5 shows the time change of currents obtained by using the sample solutions L obtained by adjusting the concentration of the residual chlorine contained in the sample solution L to 0,5 ppm, 1,0 ppm, 1,5 ppm, 2,0 ppm, 2,5 ppm and measuring current values of the sample solutions L when setting the potential of the working electrode 2 to the reference electrode 3 to + 1,1 V by the potentiostat 5.

FIG. 6 shows a calibration curve of concentration and current values when the applied voltage is +1.1 V based on the results obtained in FIG. 5. As shown in FIG. 6, the calibration curve in which the residual chlorine correlates a maximum current value well could be produced. Therefore, even a small amount (low concentration) of the residual chlorine can be correctly measured.

The residual chlorine measuring device according to the second embodiment thus constituted can measure the concentration of the residual chlorine in a lower concentration region than the first embodiment in addition to the effect of the first embodiment.

The present invention is not limited to the embodiments.

For example, although the residual chlorine measuring devices of the embodiments measure using the three-electrode method equipped with the counter electrode, the working electrode and the reference electrode, the residual chlorine measuring device may be based on a two-electrode method provided with only the working electrode and the counter electrode. Since the three-electrode method can control the absolute value of the voltage applied between the working electrode and the counter electrode, the three-electrode method can measure highly precisely and highly sensitively. However, since the two-electrode method uses only two electrodes, that is, the working electrode and the counter electrode, the constitution of the flow cell can be simplified and miniaturized. In addition, the measuring cell can be also chipped and disposed, and a simpler measurement can be performed.

Also, in the embodiments, the electrically conductive diamond electrode may take the form of a micro electrode. Herein, the diamond electrode of the micro electrode form is obtained by sharply cutting the end of a thin line made of for example, Pt or the like, making the end sharper by electrolytic polishing and then forming a thin film of a conductive diamond on the end surface.

Furthermore, although the counter electrode is the platinum (Pt) electrode in the embodiments, for example, carbon, stainless steel, gold, diamond and SnO₂ or the like can be also used.

In addition, although the reference electrode is the silver/silver chloride electrode (Ag/AgCl electrode) in the embodiments, for example, a standard hydrogen electrode, a mercury/mercury chloride electrode, a hydrogen palladium electrode or the like can be also used.

In addition, although the surface of the electrically conductive diamond electrode is hydrogen-terminated in the embodiments, the electrically conductive diamond electrode oxygen-terminated may be used.

The method for hydrogen-terminating the surface of the electrically conductive diamond electrode is not limited to the cathodic reduction treatment, and the other various methods such as a method for heating (annealing) at 700°C or more under a hydrogen atmosphere can be used.

Also, as the method for oxygen-terminating the surface of the electrically conductive diamond electrode, the electrically conductive diamond electrode can be anodized by applying the voltage of 3,0 V to the electrically conductive diamond electrode of the "as grown" state described above and by immersing the electrically conductive diamond electrode in a sulfuric acid (H₂SO₄) of 0,1 mol/l for 30 minutes. Also, in addition, the other various methods such as a treatment using oxygen plasma can be used.

Furthermore, in addition, although sodium perchlorate is used as an electrolyte which has a buffer action in each of the embodiments, the electrolyte is not limited thereto, and, for example, a phosphate buffer solution (PBS) or the like used as a buffer solution can be also used.

Furthermore, in addition, although the electrically conductive diamond electrode to which the boron is doped is used in the embodiments, an electrically conductive diamond electrode to which a group 13 element or a group 15 element such as nitrogen and phosphorus is doped may be used.

Aspects of the present invention may be further elucidated based on the following remarks:

A residual chlorine measuring method is provided, comprising steps of bringing a counter electrode 4, a working electrode 2 and a reference electrode 3 into contact with a sample solution L containing residual chlorine which is an object to be measured, applying a voltage between the counter electrode 4 and the working electrode 2, and measuring a current value under the voltage to calculate a concentration of the residual chlorine, wherein a working electrode 2 is used which is an electrically conductive diamond electrode to which a group 13 element or a group 15 element is doped, wherein a reference electrode 4 is used which is a silver/silver chloride electrode, and wherein a current value is measured when a potential of the electrically conductive diamond electrode is set to +0,5 V to +1,5 V when compared to a potential of the silver/silver chloride.

A working electrode 2 may be used which is a electrically conductive diamond electrode to which at least one kind of element selected from the group consisting of boron, nitrogen and phosphorus is doped.

An electrically conductive diamond electrode may be used having a surface which is hydrogen-terminated.

An electrically conductive diamond electrode may be used having a surface which is oxygen-terminated.

A residual chlorine measuring device is provided for measuring a residual chlorine concentration in a sample solution L, comprising a working electrode 2, a counter electrode 4, a reference electrode 3, a voltage applying part 5 for applying a voltage to the working electrode 2 and the counter electrode 4, a current measuring part for measuring a current value in the applied voltage, and an information processor 6 for calculating a residual chlorine concentration based on a current measuring signal from the current measuring part, wherein the working electrode 2 is an electrically conductive diamond electrode to which a group 13 element or a group 15 element is doped, wherein the reference electrode 3 is a silver/silver chloride electrode, and wherein the information processor 6 controls the potential of the electrically conductive diamond electrode to the silver/silver chloride electrode to +0,5 V to +1,5 V.

The working electrode 2 may be an electrically conductive diamond electrode to which at least one kind of element selected from the group consisting of boron, nitrogen and phosphorus is doped.

A surface of the electrically conductive diamond electrode may be hydrogen-terminated.

A surface of the electrically conductive diamond electrode may be oxygen-terminated.

In addition, a part or all of each of the embodiments or modification embodiments described above may be suitably combined. The present invention is not limited to each of the embodiments, and needless to say, various changes can be made within the scope of the present invention without departing the spirit.

## Claims

1. Residual chlorine measuring method,
comprising the steps of:
- bringing a counter electrode (4), a working electrode (2) and a reference electrode (3) into contact with a sample solution (L) containing residual chlorine which is an object to be measured,
- applying a voltage between the counter electrode (4) and the working electrode (2), and
- measuring a current value under the voltage to calculate a concentration of the residual chlorine,
- wherein a working electrode (2) is used which is an electrically conductive diamond electrode to which a group 13 element or a group 15 element is doped,
- wherein a reference electrode (4) is used which is a silver/silver chloride electrode, and
- wherein a current value is measured when a potential of the electrically conductive diamond electrode is set to +0,5 V to +1,5 V when compared to a potential of the silver/silver chloride.

2. Residual chlorine measuring method according to claim 1,
wherein a working electrode (2) is used which is a electrically conductive diamond electrode to which at least one kind of element selected from the group consisting of boron, nitrogen and phosphorus is doped.

3. Residual chlorine measuring method according to claim 1 or 2,
wherein an electrically conductive diamond electrode is used having a surface which is hydrogen-terminated.

4. Residual chlorine measuring method according to claim 1 or 2,
wherein an electrically conductive diamond electrode is used having a surface which is oxygen-terminated.

5. Residual chlorine measuring device,
for measuring a residual chlorine concentration in a sample solution (L), comprising:
- a working electrode (2),
- a counter electrode (4),
- a reference electrode (3),
- a voltage applying part (5) for applying a voltage to the working electrode (2) and the counter electrode (4),
- a current measuring part for measuring a current value in the applied voltage, and
- an information processor (6) for calculating a residual chlorine concentration based on a current measuring signal from the current measuring part,
- wherein the working electrode (2) is an electrically conductive diamond electrode to which a group 13 element or a group 15 element is doped,
- wherein the reference electrode (3) is a silver/silver chloride electrode, and
- wherein the information processor (6) controls the potential of the electrically conductive diamond electrode to the silver/silver chloride electrode to +0,5 V to +1,5 V.

6. Residual chlorine measuring device according to claim 5,
wherein the working electrode (2) is an electrically conductive diamond electrode to which at least one kind of element selected from the group consisting of boron, nitrogen and phosphorus is doped.

7. Residual chlorine measuring device according to claim 5 or 6,
wherein a surface of the electrically conductive diamond electrode is hydrogen-terminated.

8. Residual chlorine measuring device according to claim 5 or 6,
wherein a surface of the electrically conductive diamond electrode is oxygen-terminated.
